# EUROPEAN PATENT APPLICATION

(11) **EP 3 178 404 A1**
(43) Date of publication of application: **14.06.2017**
(21) Application number: 15829756.4
(22) Date of filing: 22.05.2015
(51) Int. Cl.: A61B 10/04, A61B 8/12

(54) **ULTRASONIC BIOPSY NEEDLE**

(30) Priority: 07.08.2014 JP 2014161577
(71) Applicant: Olympus Corporation, Tokyo 192-8507 (JP)
(72) Inventor: UEMICHI Katsuji, Tokyo 192-8507 (JP); KIKUCHI Yasuhiko, Tokyo 192-8507 (JP); SATO Yukio, Tokyo 192-8507 (JP); NAGAMATSU Ryuji, Tokyo 192-8507 (JP); ONISHI Koji, Tokyo 192-8507 (JP); SAKAMOTO Yuji, Tokyo 192-8507 (JP)
(74) Representative: Schicker, Silvia
(86) International application number: PCT/JP2015/064795
(87) International publication number: WO 2016/021278

(57) **Abstract**

An ultrasonic biopsy needle including: a sheath inserted through a channel of an ultrasonic endoscope; a needle tube which is inserted through the sheath and capable of advancing and retracting in the sheath; and an operation portion which is connected to a proximal end of the sheath and is provided to advance and retract the needle tube. The sheath includes a meandering suppressing portion that is positioned more proximal than a proximal end of a bending portion of the ultrasonic endoscope when a distal end of the sheath is positioned in a visual field of an optical image-capturing mechanism of the ultrasonic endoscope. The meandering suppressing portion is formed of a metal coil, the ratio of the inner diameter of the channel to the outer diameter of the coil is in a range from 1.0:0.66 to 1.0:0.84, and the initial tension of the coil is equal to or greater than 5.0 [N].

## Description

### [Technical Field]

The present invention relates to a biopsy needle used together with an ultrasonic endoscope.

Priority is claimed on Japanese Patent Application No. 2014-161577, filed on August 7, 2014, the contents of which are incorporated herein by reference.

### [Background Art]

In the related art, an inspection method referred to as a biopsy, in which a very small amount of body tissue is collected and is observed via a microscope, has been known. Since it is difficult for an operator to observe a tissue in a deep site such as an organ via an optical endoscope when collecting the tissue, the operator acquires an ultrasonic tomographic image of the organ via an ultrasonic endoscope or the like, incises the organ via a biopsy needle under ultrasonic observation, and acquires the tissue. In order to accurately acquire an ultrasonic image of a tissue via an ultrasonic endoscope, an operator is required to reliably bring an ultrasonic vibrator of the ultrasonic endoscope into contact with a target tissue.

PTL 1 discloses a puncture needle which can be used together with an ultrasonic endoscope. PTL 2 discloses a puncture needle in which a metal blade is coated with resin so as to decrease flexibility of a tube into which the puncture needle is inserted, and reduce the coefficient of extension and contraction.

### [Citation List]

### [Patent Literature]

[PTL 1] Japanese Unexamined Patent Application, First Publication No. 2001-120557
[PTL 2] Japanese Unexamined Patent Application, First Publication No. H9-131399

### [Summary of Invention]

### [Technical Problem]

A respiratory endoscope has a thinner diameter than that of an endoscope for digestive organs. For this reason, the amount of bending force of a bending portion, which is actively bent in the respiratory endoscope, is smaller than that of a bending portion of the digestive endoscope. A sheath of a biopsy needle used in the respiratory endoscope is required to have flexibility to prevent a decrease in the bending capability of the respiratory endoscope when the biopsy needle is inserted into the respiratory endoscope.

In contrast, since a highly flexible sheath typically has good extending and contracting properties, limiting of the extension of the sheath and improving the flexibility of the sheath become difficult in an ultrasonic biopsy needle used in a respiratory endoscope which has a thinner diameter and is not stiff. In a case where the highly flexible sheath extends when the biopsy needle punctures a tissue, the sheath protrudes from a distal end of the endoscope by the amount of extension, and the protruding sheath pushes the tissue. In this case, since the distance between the tissue and the ultrasonic vibrator is increased, the ultrasonic image becomes unclear.

The present invention is made in light of these problems, and an object of the present invention is to realize both of limiting the extension of a sheath caused by the advancement and retraction of a needle tube, and improving flexibility of the sheath.

### [Solution to Problem]

According to a first aspect of the present invention, an ultrasonic biopsy needle includes a sheath inserted through a channel of an ultrasonic endoscope; a needle tube which is inserted through the sheath and can capable of advancing and retracting in the sheath; and an operation portion which is connected to a proximal end of the sheath and is provided to advance and retract the needle tube. The sheath includes a meandering suppressing portion that is positioned more proximal than a proximal end of a bending portion of the ultrasonic endoscope when a distal end of the sheath is positioned in a visual field of an optical image-capturing mechanism of the ultrasonic endoscope. The meandering suppressing portion is formed of a metal coil, the ratio of the inner diameter of the channel to the outer diameter of the coil is in a range from 1.0:0.66 to 1.0:0.84, and the initial tension of the coil is equal to or greater than 5.0 [N].

According to a second aspect of the present invention, in the ultrasonic biopsy needle according to the first aspect, the sheath may be formed of the coil in the entire length of the sheath.

According to a third aspect of the present invention, in the ultrasonic biopsy needle according to the first aspect, the coil may include a metal wire, and a resin coating with which the metal wire is coated.

According to a fourth aspect of the present invention, in the ultrasonic biopsy needle according to the third aspect, the spring constant of the coil may be 0.7 N/mm.

According to a fifth aspect of the present invention, in the ultrasonic biopsy needle according to the first aspect, the channel may have an inner diameter of equal to or greater than 2.0 mm and equal to or less than 2.2 mm. A sloped portion may be formed in a distal end side of the channel such that the angle of the sloped portion is fixed in order for the ultrasonic biopsy needle to protrude from the channel while being inclined with respect to a vibrator of the ultrasonic endoscope. The maximum amount of movement of the needle tube with respect to the sheath may be equal to or greater than 5% of the total length of the sheath.

### [Advantageous Effects of Invention]

In the ultrasonic biopsy needle according to each of the aspects, the extension of the sheath caused by the forward and backward movement of the needle tube can be limited, and flexibility of the sheath can be improved.

### [Brief Description of Drawings]

FIG. 1 is a view showing the entirety of a biopsy system including an ultrasonic biopsy needle in an embodiment of the present invention.
FIG. 2 is a sectional view of a distal end portion of an ultrasonic endoscope which is assembled with the ultrasonic biopsy needle in the embodiment of the present invention.
FIG. 3 is a sectional view of a distal end portion of the ultrasonic biopsy needle in the embodiment of the present invention.
FIG. 4 is a sectional view showing a state in which the ultrasonic biopsy needle in the embodiment of the present invention is mounted into the ultrasonic endoscope.
FIG. 5 is a partial sectional view of the ultrasonic biopsy needle in the embodiment of the present invention.
FIG. 6 is a view showing an operation portion of the ultrasonic biopsy needle in the embodiment of the present invention.
FIG. 7 is a perspective view showing a state in which the ultrasonic biopsy needle in the embodiment of the present invention is mounted into the ultrasonic endoscope.
FIG. 8 is a view showing the operation of the ultrasonic biopsy needle in the embodiment of the present invention.
FIG. 9 is a view showing the operation of the ultrasonic biopsy needle in the embodiment of the present invention.
FIG. 10 is a view showing the operation of the ultrasonic biopsy needle in the embodiment of the present invention.
FIG. 11 is a graph showing a comparison between percent defectives in Example 1, Example 2, and Comparative Example 1 of the present invention.
FIG. 12 is a graph showing a comparison between the amounts of extension of a sheath in Example 1, Example 2, and Comparative Example 1 of the present invention.

### [Description of Embodiments]

### (First Embodiment)

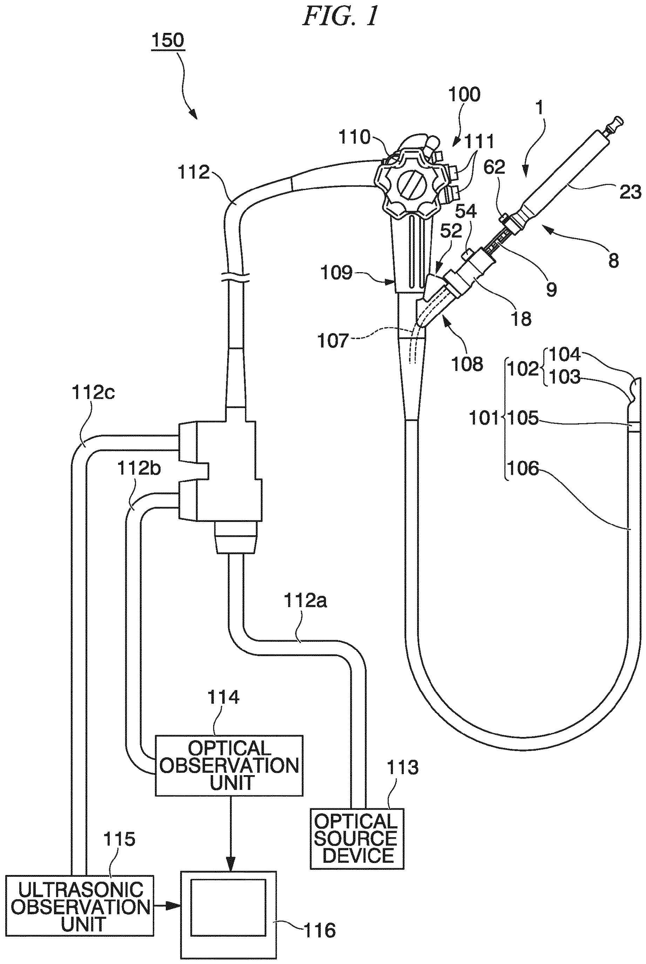

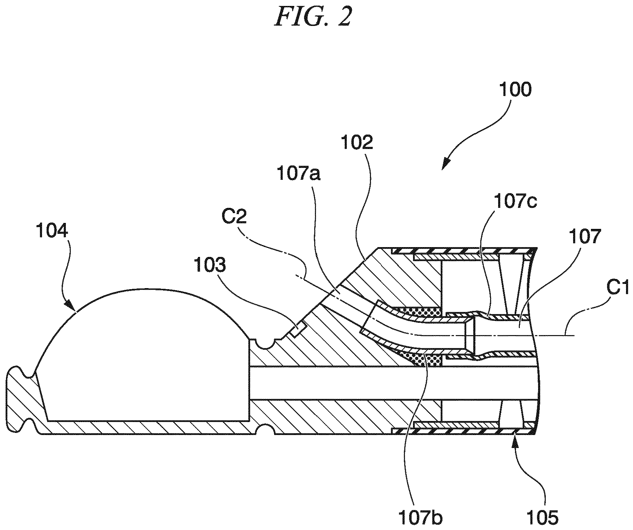

An embodiment of the present invention will be described. FIG. 1 is a schematic view showing the configuration of a biopsy system 150 in the embodiment including a biopsy needle 1 and an ultrasonic endoscope 100. FIG. 2 is a sectional view of a distal end portion of the ultrasonic endoscope 100 which is an endoscope of the biopsy system 150.

The ultrasonic biopsy needle 1 (hereinafter, simply referred to as a "biopsy needle 1") in the embodiment shown in FIG. 1 is a portion of the biopsy system 150, and is a puncture needle used for biopsy in combination with the ultrasonic endoscope 100.

First, an example of an endoscope, which is used together with the biopsy needle 1 in the embodiment, will be described. The configuration of an endoscope useable together with the biopsy needle in the embodiment is not limited to a specific configuration.

The ultrasonic endoscope 100 exemplified in the embodiment is a thin-diameter endoscope assumed to be used to diagnose or treat a respiratory organ. The ultrasonic endoscope 100 includes an inserting portion 101; an operation portion 109; a universal cord 112; an optical source device 113; an optical observation unit 114; and an ultrasonic observation unit 115. The inserting portion 101 is inserted into a human body from a distal end of the inserting portion 101. The operation portion 109 is mounted to a proximal end of the inserting portion 101. One end of the universal cord 112 is connected to a side portion of the operation portion 109. The optical source device 113 is connected to the other end of the universal cord 112 via a branch cable 112a. The optical observation unit 114 is connected to the other end of the universal cord 112 via a branch cable 112b. The ultrasonic observation unit 115 is connected to the other end of the universal cord 112 via a branch cable 112c.

A distal end rigid portion 102, a bending portion 105, and a flexible tubular portion 106 are provided side by side in the inserting portion 101 sequentially from a distal end side of the inserting portion 101.

The distal end rigid portion 102 includes an optical image-capturing mechanism 103 for optical observation, and an ultrasonic scanning mechanism 104 for ultrasonic observation.

The optical image-capturing mechanism 103 includes various configuration elements such as an image-capturing optical system, an image sensor (for example, a CCD or a CMOS), and a CPU. The visual field of the image-capturing optical system is diagonally oriented to a front side of the distal end rigid portion 102. The image sensor detects an image of an object which is incident thereto via the image-capturing optical system. The CPU controls the operation of the image sensor.

The ultrasonic scanning mechanism (probe) 104 includes an ultrasonic vibrator (not shown) emitting and receiving an ultrasonic wave. An ultrasonic wave emitted by the ultrasonic vibrator collides with and is reflected from an observation target, and the ultrasonic vibration receives the reflected wave. The ultrasonic scanning mechanism 104 outputs a signal to the ultrasonic observation unit 115 based on the ultrasonic wave received by the ultrasonic vibrator. The ultrasonic scanning mechanism 104 in the embodiment is used to acquire an ultrasonic image of a tissue which is a biopsy target, and an ultrasonic image of a needle tube 3 during a biopsy procedure.

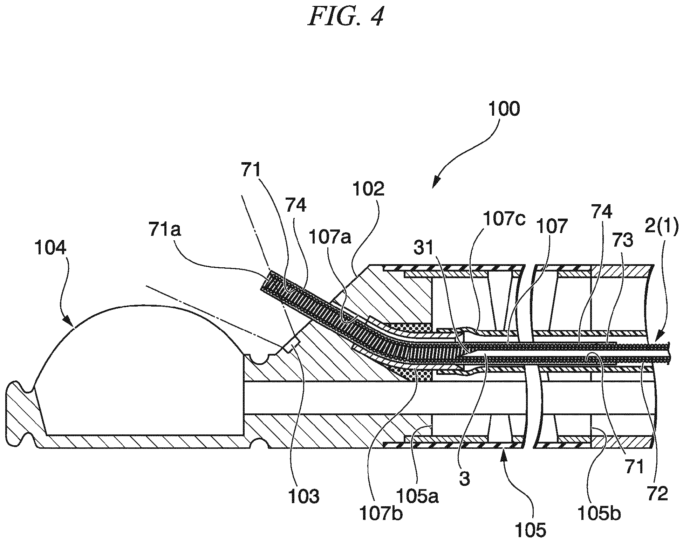

The bending portion 105 is formed into a cylindrical shape, and can be bent in a predetermined direction by pulling an angle wire (not shown) (fixed to a distal end 105a (refer to FIG. 4) of the bending portion 105, and extending to the operation portion 109) via the operation portion 109. The bending portion 105 in the embodiment can be bent in two directions along a scanning direction of an ultrasonic wave.

In the embodiment, an endoscope which includes an inserting portion having a thin outer diameter and can be bent in two directions is used to treat a respiratory organ; however, an endoscope which has a thick outer diameter but offers a high degree of freedom in operation and can be bent in four directions may be used to treat a digestive organ.

The flexible tubular portion 106 is a cylindrical flexible member capable of guiding the distal end rigid portion 102 to a desired position in a luminal tissue or a body cavity.

A channel 107, and a tubular path (not shown) through which air or water is blown and suctioned, are provided inside the bending portion 105 and the flexible tubular portion 106.

The channel 107 shown in FIGS. 1 and 2 is a cylindrical portion into which the biopsy needle 1 is inserted.

One end of the channel 107 opens in the vicinity of a distal end portion of the distal end rigid portion 102, and the other end of the channel 107 opens in a side surface of the operation portion 109 on a distal end side. A flange-shaped proximal end connector 108 is fixed to the other end of the channel 107. The biopsy needle 1 used together with the ultrasonic endoscope 100 can be fixed to the proximal end connector 108. The channel 107 in the embodiment has an inner diameter of equal to or greater than 2.0 mm and equal to and less than 2.2 mm. The channel 107 in the embodiment has an inner diameter smaller than the inner diameter of a channel of an endoscope for a digestive organ.

As shown in Fig. 2, the channel 107 includes a sloped portion 107a; an angled portion 107b; and a channel tube 107c. The sloped portion 107a is inclined with respect to an axial line C1 of the inserting portion 101 in the distal end rigid portion 102. The angled portion 107b is connected to a proximal end of the sloped portion 107a. The channel tube 107c is connected to a proximal end of the angled portion 107b.

The sloped portion 107a is provided in the distal end rigid portion 102 such that a through hole, which has a straight central axis line inclined with respect to the axial line C1 of the inserting portion 101, is formed in the distal end rigid portion 102. The position of a central axis line C2 of the through hole formed in the sloped portion 107a is included in a scanning surface of the ultrasonic scanning mechanism 104. For this reason, when the biopsy needle 1 is inserted into the sloped portion 107a, the sloped portion 107a is capable of guiding the needle tube 3 (refer to FIG. 4) of the biopsy needle 1 to the scanning surface, and enables the needle tube 3 to protrude while being inclined with respect to the vibrator of the ultrasonic scanning mechanism 104.

The inclination angle of the central axis line C2 of the sloped portion 107a with respect to the axial line C1 of the inserting portion 101 may be appropriately set to correspond to a portion or the like which is a treatment target. In the embodiment, the needle tube 3 protrudes at the inclination angle (for example, angle of 23° or greater and 28° or less) of the central axis line C2 of the sloped portion 107a with respect to the axial line C 1 of the inserting portion 101 such that an ultrasonic image of the needle tube 3 can be acquired.

The angled portion 107b is a tube that is curved or bent at a predetermined angle. The angled portion 107b is capable of changing the direction of a distal end of the biopsy needle 1, which is guided from the channel tube 107c to the sloped portion 107a, to a direction along the central axis line C2 of the sloped portion 107a. The sloped portion 107a and the channel tube 107c are connected to each other via the angled portion 107b. In the embodiment, the angled portion 107b has an arc shape that is bent at a predetermined radius of curvature.

The channel tube 107c opens in the vicinity of a proximal end of the distal end rigid portion 102 so as to face a distal end side of the inserting portion 101 in a direction parallel to the axial line C1 of the inserting portion 101. The channel tube 107c extends to a proximal end side of the inserting portion 101 while being parallel to the axial line C1 of the inserting portion 101, and is fixed to the proximal end connector 108.

The operation portion 109 shown in FIG. 1 includes an outer surface which is formed such that an operator, a user of the ultrasonic endoscope 100, can hold the operation portion 109 with a hand. The operation portion 109 includes a bending operation mechanism 110 and multiple switches 111. The bending operation mechanism 110 is capable of bending the bending portion 105 by pulling the angle wire. Air or water is blown or suctioned through the tubular path by operating the multiple switches 111.

The optical source device 113 is a device emitting illumination light required for the optical image-capturing mechanism 103 to capture an image of a target.

The optical observation unit 114 is configured to project an image which is captured by the image sensor of the optical image-capturing mechanism 103 on a monitor 116.

The ultrasonic observation unit 115 receives a signal output from the ultrasonic scanning mechanism 104, generates an image based on the received signal, and projects the generated image on the monitor 116.

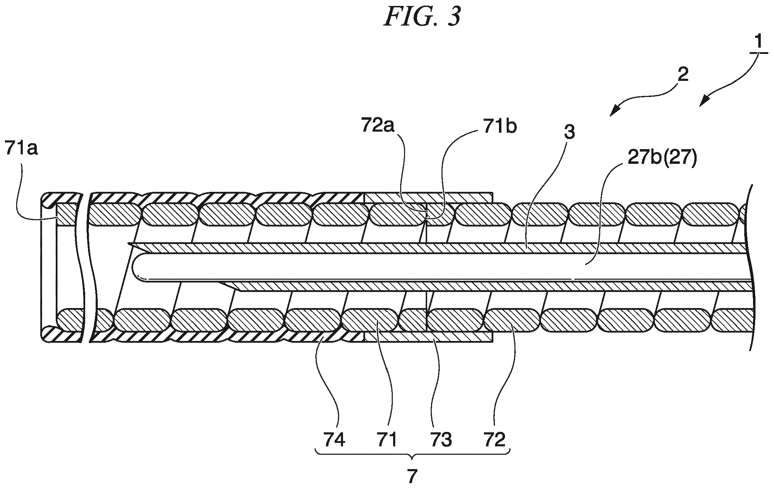

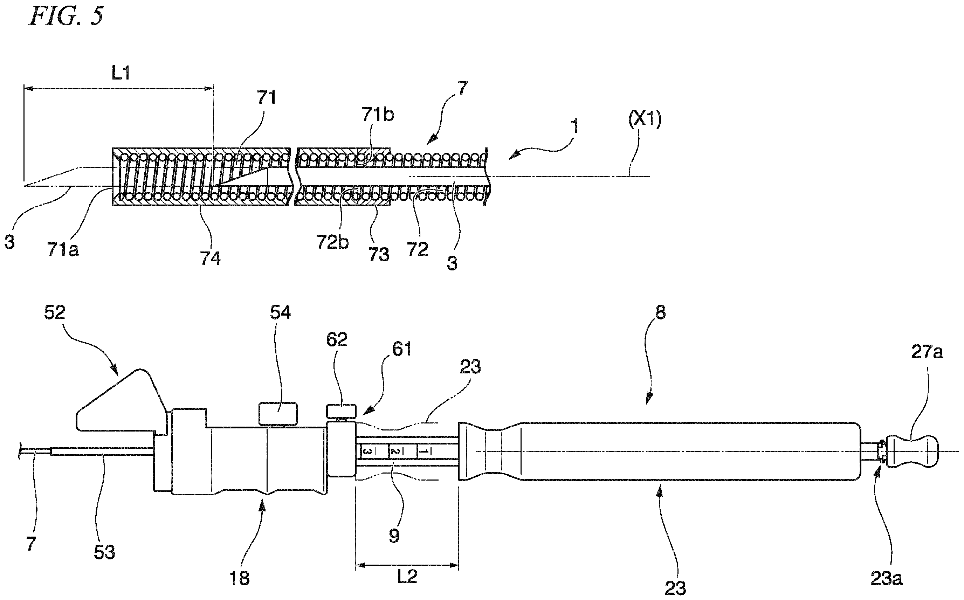

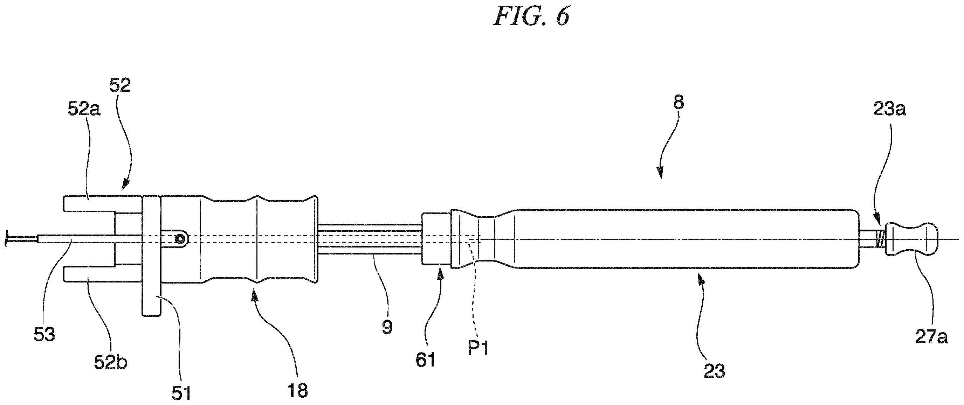

Hereinafter, the configuration of the biopsy needle 1 will be described. FIG. 3 is a sectional view of a distal end portion of the biopsy needle 1. FIG. 4 is a sectional view showing a state in which the biopsy needle 1 is mounted into the ultrasonic endoscope 100. FIG. 5 is a partial sectional view of the biopsy needle 1. FIG. 6 is a view showing an operation portion 8 of the biopsy needle 1.

As shown in FIGS. 1 and 3, the biopsy needle 1 includes an inserting body 2 inserted into a human body; the operation portion (treatment tool operating portion) 8 for operating the inserting body 2; and a stylet (core bar) 27.

The inserting body 2 is a long member which can be mounted into the channel 107 so as to be able to protrude from the distal end of the inserting portion 101 of the ultrasonic endoscope 100. The inserting body 2 includes the needle tube 3, and a cylindrical sheath 7 into which the needle tube 3 is inserted.

The needle tube 3 is a cylindrical member with a 22-gauge size which is moved forward and backward by the operation portion 8.

The needle tube 3 may be made of a material which has flexibility and elasticity by which the needle tube 3 is easily restored to its straight state even if the needle tube 3 is bent by an external force. For example, alloy materials such as a stainless alloy, a nickel-titanium alloy, and a cobalt-chromium alloy can be adopted as the material of the needle tube 3.

An opening 31 is formed at a distal end of the needle tube 3. The opening 31 is sharpened so as to enable the needle tube 3 to puncture a tissue, and the tissue is suctioned into the needle tube 3 through the opening 31.

The opening 31 provided at the distal end of the needle tube 3 is formed by cutting a distal end of a tubular member (which forms the needle tube) off diagonally to an axial line X1 of the tubular member. The opening 31 is sharply formed to be able to incise a biological tissue. The specific shape of the opening 31 may be appropriately selected from various well-known shapes while a tissue which is a target is taken into consideration.

As shown in FIG. 3, the sheath 7 includes a distal end coil 71 forming a distal end portion of the sheath 7; a proximal end coil 72 forming a proximal end portion of the sheath 7; a connecting portion 73; and a resin coating 74.

The distal end coil 71 is made of a metal wire having a rectangular section, and is formed into a coil shape. The metal wire of the distal end coil 71 has a rectangular sectional shape having a thickness of 0.20 mm ± 0.01 mm and a width of 0.65 mm.

The distal end coil 71 has an inner diameter φ of 1.10 mm. The ratio of the inner diameter of the channel 107 to the outer diameter of the distal end coil 71 is in a range from 1.0:0.84 to 1.0:0.96. The initial tension of the distal end coil 71 is equal to or greater than 2.0 [N].

The distal end coil 71 is an extension limiting portion limiting the extension of the distal end portion of the sheath 7 in a direction of the central axis line of the sheath 7.

The proximal end coil 72 is made of a metal wire having a rectangular section, and is formed into a coil shape. The metal wire of the proximal end coil 72 has a rectangular sectional shape having a thickness of 0.23 mm ± 0.01 mm and a width of 0.60 mm.

The proximal end coil 72 has an inner diameter φ of 1.10 mm. The ratio of the inner diameter of the channel 107 to the outer diameter of the proximal end coil 72 is in a range from 1.0:0.66 to 1.0:0.84. The initial tension of the proximal end coil 72 is equal to or greater than 5.0 [N].

The proximal end coil 72 is a meandering suppressing portion suppressing the meandering of the proximal end portion of the sheath 7.

The dimension of the sheath 7 and other specific design dimensions given in the embodiment are merely specific examples.

The connecting portion 73 is a cylindrical member through which the distal end coil 71 is connected to the proximal end coil 72. The connecting portion 73 is fixed to a proximal end portion of the distal end coil 71 and to a distal end portion of the proximal end coil 72 by brazing.

The resin coating 74 is mounted to the distal end coil 71 so as to cover the outer circumference of the distal end coil 71. The resin coating 74 has a thickness of 0.15 mm or greater and 0.2 mm or less. The resin coating 74 is formed of a heat-contractible tube that covers and comes into close contact with the distal end coil 71 after the distal end coil 71 is formed.

The inner surface of the resin coating 74 is formed to conform to the outer surface shape of the distal end coil 71. In the embodiment, since the distal end coil 71 is wound in a coil shape, the inner surface of the resin coating 74 is not jammed into a gap of an adjacent portion.

The inner surface of the resin coating 74 is not fixed to the distal end coil 71. That is, the resin coating 74 is engaged with the outer surface of the distal end coil 71 due to a contracting force causing the resin coating 74 to be smaller than the outer diameter of the distal end coil 71. Accordingly, when the distal end coil 71 is bent and deformed, a portion of the inner surface of the resin coating 74 moves away from the outer surface of the metal wire of the distal end coil 71 such that the portion of the inner surface can extend and contract.

The outer surface of the resin coating 74 is concave and convex to correspond to the outer surface shape of the distal end coil 71. The outer surface shape of the resin coating 74 is not limited to a specific shape.

The resin coating 74 is provided on the distal end coil 71, and thus, the outer diameter of the distal end coil 71 of the sheath 7 is in a range of equal to or greater than φ 1.7 mm and equal to or less than φ 1.9 mm.

As shown in FIGS. 5 and 6, the operation portion 8 includes an operation body 9; a sheath adjuster 18 provided on a distal end side of the operation body 9; and a needle slider 23 provided on a proximal end side of the operation body 9.

The operation body 9 is made of ABS resin or the like, and has a tubular cavity into which the needle tube 3 and the sheath 7 can be inserted. The distal end side of the operation body 9 is inserted into the tubular sheath adjuster 18. The proximal end side of the operation body 9 is inserted into the tubular needle slider 23. Axial slide motions between the operation body 9 and the sheath adjuster 18, and between the operation body 9 and the needle slider 23 are allowed while relative rotation around an axial line therebetween is suppressed due to engagement between grooves, convex portions, or the like (not shown) formed on the outer surfaces.

A slider lock 51 is provided in a distal end portion of the sheath adjuster 18 in such a way that the slider lock 51 can be attached to and detached from the proximal end connector 108 of the ultrasonic endoscope 100. The slider lock 51 is slid in a direction perpendicular to an axial line of the operation portion 8, and is engaged with the proximal end connector 108 such that the operation portion 8 can be fixed to the ultrasonic endoscope 100. A holder (fixing portion) 52 including a pair of wall portions 52a and 52b is provided on a distal end side of the slider lock 51. The holder 52 is fixed with respect to the sheath adjuster 18. The pair of wall portions 52a and 52b of the holder 52 are substantially parallel to each other. The distance between the pair of wall portions 52a and 52b is set to a value to the extent that the wobbling of a distal end side of the operation portion 109 of the ultrasonic endoscope 100 can be absorbed.

A stainless-steel support pipe 53 protrudes from the distal end portion of the sheath adjuster 18. When the biopsy needle 1 is mounted into the ultrasonic endoscope 100, a distal end portion of the support pipe 53 is inserted into the channel 107. The support pipe 53 is inserted into the operation body 9. When the needle slider 23 is moved to its most backward position with respect to the operation body 9, a proximal end of the support pipe 53 is positioned (for example, disposed at a position P1 shown in FIG. 6) more proximal than a distal end of the needle slider 23. The sheath 7 is inserted into the support pipe 53. The proximal end portion of the sheath 7 protrudes from the proximal end of the support pipe 53, and is fixed to the operation body 9 by bonding or the like.

A fixing screw 54 is mounted to the sheath adjuster 18. The fixing screw 54 passes through the sheath adjuster 18, and is fitted into a screw hole (not shown) provided in the operation body 9. When the fixing screw 54 is tightened to the operation body 9, the sheath adjuster 18 is pressed against and is brought into contact with the operation body 9 such that the sheath adjuster 18 and the operation body 9 can be non-slidably fixed together. It is possible to adjust the length of protrusion of the sheath 7 from the channel 107 when the operation portion 8 is fixed to the ultrasonic endoscope 100 by changing a positional relationship between the sheath adjuster 18 and the operation body 9, and it is possible to fix the length of protrusion using the fixing screw 54.

As shown in FIG. 1, an axial line of the fixing screw 54 may be disposed to be aligned with an axial line of the operation portion 109 accommodated in the holder 52. Accordingly, since the fixing screw 54 is not biased in a rightward and leftward direction when the operation portion 8 is positioned to face the front, an operator can easily operate the fixing screw 54 regardless of the dominant hand. When the axial line of the fixing screw 54 is aligned with the axial line of the operation portion 109 accommodated in the holder 52, even if the fixing screw 54 is mounted toward a direction opposite to the direction shown in FIG. 1, it is possible to obtain substantially the same effects.

An outer circumferential surface of the distal end portion of the sheath adjuster 18 has concavities and convexities such that an operator can easily grasp the sheath adjuster 18.

The needle slider 23 is fixed to a proximal end of the needle tube 3. The needle slider 23 is connected to the operation body 9 in such a way that the needle slider 23 can be moved with respect to the operation body 9.

A proximal end side of the needle tube 3 protrudes from a proximal end of the sheath 7, and is fixed to the needle slider 23. For this reason, when the needle slider 23 is slid with respect to the operation body 9, the needle tube 3 is capable of protruding from and retracting into the distal end of the sheath 7. A stopper 61 is mounted on a distal end side of the needle slider 23 in such a way that the stopper 61 can be moved with respect to the operation body 9. The stopper 61 includes a fixing screw 62. It is possible to fix the stopper 61 to the operation body 9 by tightening the fixing screw 62. As shown in FIG. 1, an axial line of the fixing screw 62 may be disposed to be aligned with the axial line of the operation portion 109 accommodated in the holder 52. Accordingly, since the fixing screw 62 is not biased in the rightward and leftward direction when the operation portion 8 is positioned to face the front, an operator can easily operate the fixing screw 62 regardless of the dominant hand. When the axial line of the fixing screw 62 is aligned with the axial line of the operation portion 109 accommodated in the holder 52, even if the fixing screw 62 is mounted toward the direction opposite to the direction shown in FIG. 1, it is possible to obtain substantially the same effects.

The fixing screw 62 and the fixing screw 54 may be disposed in the same direction, or the fixing screw 62 may be disposed in a direction opposite to where the fixing screw 54 is disposed.

As shown in FIG. 5, since the needle slider 23 can be moved forward with respect to the operation body 9 only up to a position in which the needle slider 23 comes into contact with the stopper 61, it is possible to adjust the maximum length of protrusion of the needle tube 3 from the sheath 7 by adjusting the fixing position of the stopper 61 with respect to the operation body 9. In the embodiment, an operation stroke length (the maximum amount of movement) L2 of the needle tube 3 obtained by operating the needle slider 23 is a length greater than or equal to 5% of the total length of the sheath 7. The operation stroke length L2 is affected by the position of a treatment target site, and in the embodiment, the operation stroke length L2 of the needle tube 3 obtained by operating the needle slider 23 may be 40 mm or greater.

A state in which the needle slider 23 is moved to and present at a limit position on the proximal end side of the operation body 9 is an initial state before an operator starts to use the biopsy needle 1. In the initial state, the distal end of the needle tube 3 is positioned inside the sheath 7. More specifically, in the initial state, the distal end of the needle tube 3 is positioned inside the distal end coil 71. When the sheath 7 and the channel 107 have a positional relationship (refer to FIG. 4) in which the sheath 7 is mounted into the channel 107 of the ultrasonic endoscope 100, and the distal end portion of the sheath 7 can be optically observed using the ultrasonic endoscope 100, the distal end of the needle tube 3 is more proximal than the distal end 105a of the bending portion 105.

In the initial state, the position of the distal end of the needle tube 3 with respect to the sheath 7 is affected and changed by extension or contraction of the sheath 7 and extension or contraction of the needle tube 3. A change in the position of the distal end of the needle tube 3 with respect to the sheath 7 is affected by temperature, humidity, the state of the mounting of the needle tube 3 into the channel 107 of the ultrasonic endoscope 100, the amount of operation force applied to the biopsy needle 1, and the like.

In a process in which the inserting body 2 is inserted into the channel 107 (refer to FIG. 4), the proximal end coil 72 receives a compressive force in a direction of a center axis line of the proximal end coil 72 such that the proximal end coil 72 meanders with respect to a center axis line of the needle tube 3. In a case where the proximal end coil 72 meanders inside the channel 107, when a distal end of the inserting body 2 is guided to the distal end of the channel 107, the distal end of the needle tube 3 is positioned in the vicinity of the distal end of the sheath 7 compared to when the inserting body 2 is not inserted into the channel 107.

In the embodiment, as shown in FIG. 5, the distal end of the needle tube 3 is set to always be positioned inside the distal end coil 71 in the initial state under an environment in which a technique of using the biopsy needle 1 is assumed to be performed, considering temperature, humidity, a state of the mounting of the needle tube 3 into the channel 107 of the ultrasonic endoscope 100, and the amount of operation force applied to the biopsy needle 1.

The amount of movement of the needle slider 23 with respect to the operation body 9 substantially corresponds to the amount of movement of the distal end of the needle tube 3 with respect to the sheath 7 (refer to FIG. 5). That is, since the needle slider 23 moves the needle tube 3 with respect to the sheath 7, the amount of movement (relative stroke length L1) of the distal end of the needle tube 3 with respect to the sheath 7 is equivalent to an actual amount of movement (the operation stroke length L2) of the needle slider 23 plus an extension or a contraction of the needle tube 3. The extension or the contraction of the needle tube 3 is affected by extending and contracting properties

(elasticity) of the needle tube 3, the magnitude of frictional resistance between the needle tube 3 and the sheath 7, a state of meandering of the sheath 7 inside the channel 107, and a state of meandering of the needle tube 3 inside the sheath 7.

When the needle slider 23 is moved to and present at a limit position on the distal end side of the operation body 9, the distal end of the needle tube 3 protrudes from the distal end of the sheath 7. The length of protrusion of the needle tube 3, when the needle slider 23 is moved to and present at the limit position on the distal end side of the operation body 9, is less than the operation stroke length L2 of the needle slider 23, and may be at least 40 mm.

An opening 23a is provided in a proximal end portion of the needle slider 23, and the stylet 27 can be inserted into the needle tube 3 through the proximal end of the needle tube 3. Screw threads are provided in the opening 23a, and a well-known syringe or the like can be connected to the opening 23a. An outer circumferential surface of the distal end portion of the needle slider 23 has concavities and convexities such that an operator can easily grasp the needle slider 23.

The stylet 27 shown in FIGS. 3 and 5 includes a knob 27a that can be mounted to the opening 23 a of the needle slider 23, and a core 27b fixed to the knob 27a.

The core 27b has a sectional shape corresponding to an inner surface shape of the needle tube 3. In the embodiment, the core 27b has a circular sectional shape.

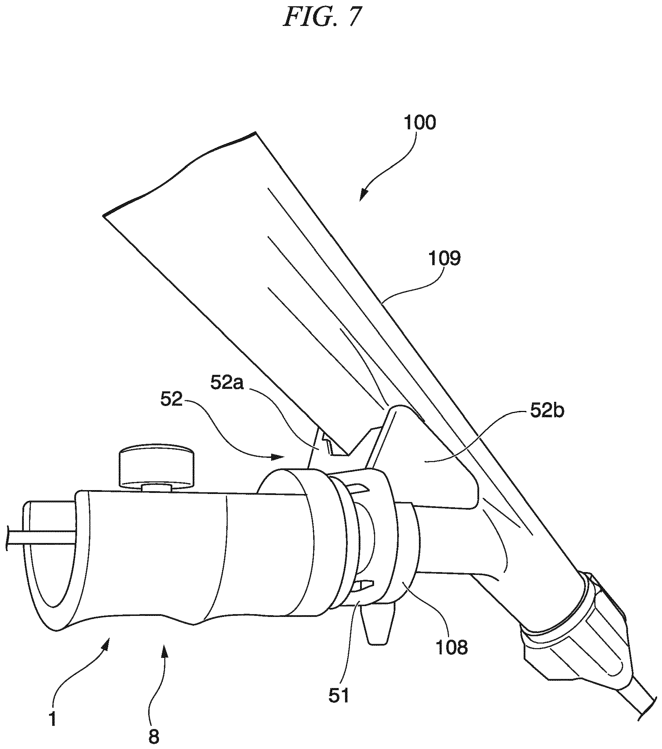

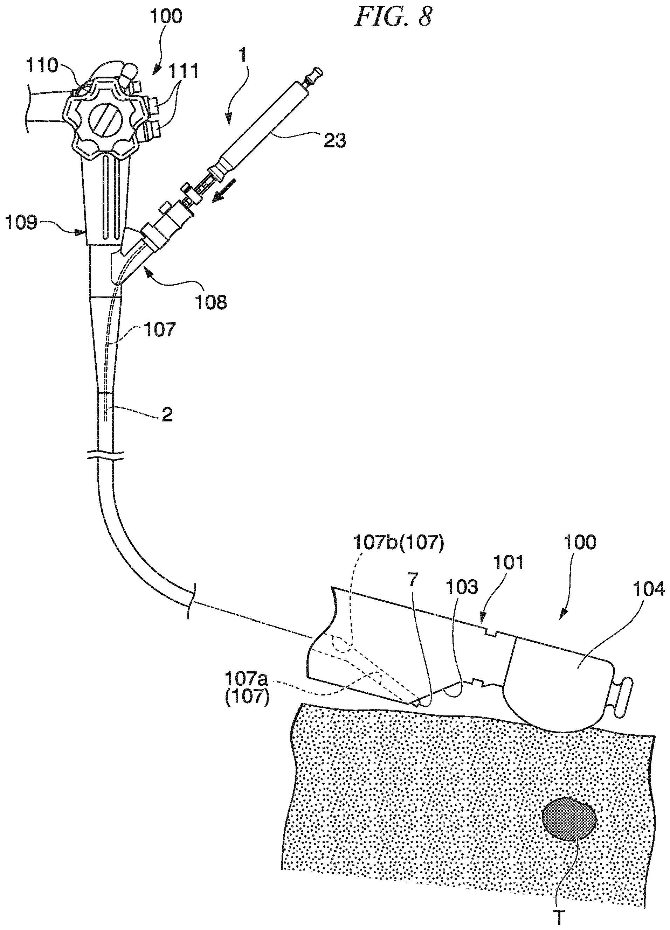

The operation of the biopsy needle 1 with the aforementioned configuration in use will be described. FIG. 7 is a perspective view showing a state in which the biopsy needle 1 is mounted into the ultrasonic endoscope 100. FIGS. 8 to 10 are views showing the operation of the biopsy needle 1.

Hereinafter, biopsy treatment, in which an operator incises a lesion (that is, a target tissue) positioned at a deep site of a lung using the needle tube 3 of the biopsy needle 1, and collects cells of the lesion through the inside of the needle tube 3, will be exemplarily described.

First, the operator inserts the inserting portion 101 of the ultrasonic endoscope 100 shown in FIG. 1 into a human body, appropriately bends the bending portion 105, and guides a distal end portion of the inserting portion 101 to the vicinity of a target tissue while observing the target tissue via the optical image-capturing mechanism 103. After guiding the distal end portion of the inserting portion 101 to the target tissue, the operator determines a site for biopsy based on results of observation via the optical image-capturing mechanism 103 and the ultrasonic scanning mechanism 104.

Subsequently, the operator inserts the inserting body 2 of the biopsy needle 1 into the channel 107 from a distal end side of the inserting body 2 through the proximal end connector 108 provided in the operation portion 109 of the ultrasonic endoscope 100. As shown in FIG. 7, the operator engages the slider lock 51, which is provided in the operation portion 8 of the biopsy needle 1, with the proximal end connector 108 while putting the distal end side of the operation portion 109 into the gap between the pair of wall portions 52a and 52b of the holder 52. Accordingly, the operation portion 8 of the biopsy needle 1 is fixed to the ultrasonic endoscope 100 such that the operation portion 8 is not rotated with respect to the operation portion 109.

At this time, the distal end of the needle tube 3 is positioned inside the distal end coil 71. Since the resin coating 74 is in close contact with the distal end coil 71, and an initial tension of 2.0 N or greater is applied to the distal end coil 71, the distal end coil 71 is held in a substantially tightly wound state. When the distal end coil 71 is deformed to conform to the bent shape of the channel 107, portions of spirals of the metal wire of the distal end coil 71 move away from each other, and the resin coating 74 extends to conform to the bent deformation of the distal end coil 71. The resin coating 74 is in close contact with the wire of the distal end coil 71, thereby enabling the limitation of variations in the gap between the spirals of the wire of the distal end coil 71 to a low level.

The needle tube 3 guided inside the channel 107 along the bent flexible tubular portion 106 is protected such that the distal end of the needle tube 3 does not penetrate through the distal end coil 71. Due to frictional resistance between an inner surface of the channel 107 and the outermost surface of the inserting body 2, the amount of contraction or meandering of the sheath 7 caused by compression is accumulated as the inserting body 2 is pushed into the channel 107. For example, sliding resistance of the sheath 7 with respect to the inner surface of the channel 107 is large in a region from the angled portion 107b to the sloped portion 107a of the channel 107. When the sheath 7 is pushed to the distal end side from a position closer to the proximal end side than the angled portion 107b, the amount of meandering of the sheath 7 may be accumulated in a region between the proximal end connector 108 and the angled portion 107b.

In the embodiment, with regard to the proximal end coil 72 of the sheath 7, the ratio of the inner diameter of the channel 107 to the outer diameter of the proximal end coil 72 is in a range of equal to or greater than 1.0:0.66 and equal to or less than 1.0:0.84, and the initial tension of the proximal end coil 72 is equal to or greater than 5.0 N. For this reason, the meandering of the sheath 7 inside the channel 107 is suppressed, and the amount of meandering of the sheath 7 is accumulated less in the region between the proximal end connector 108 and the angled portion 107b, compared to the related art. That is, in the embodiment, the proximal end coil 72 of the sheath 7 is a meandering suppressing portion that further suppresses the meandering of the sheath 7 inside the channel 107 compared to the related art. When the amount of meandering of the sheath 7 is accumulated in the region between the proximal end connector 108 and the angled portion 107b, and then the meandering is released, the sheath 7 is moved to the distal end side relative to the needle tube 3.

Since the accumulation of the amount of meandering of the sheath 7 and the releasing of meandering are repeated in a process in which the inserting body 2 is inserted into the channel 107, the degree of accumulation of the amount of meandering when the sheath 7 protrudes from the distal end of the channel 107 is not constant. The forward and backward movement of the needle tube 3 is one occasion in which the meandering of the sheath 7 is released.

Subsequently, the operator untightens the fixing screw 54, and as shown in FIG. 8, slides the sheath adjuster 18 relative to the operation body 9 and appropriately adjusts the amount of protrusion of the sheath 7 from the distal end of the inserting portion 101 of the ultrasonic endoscope 100 while observing the sheath 7 and the inside of the human body via the optical image-capturing mechanism 103 and the ultrasonic scanning mechanism 104. After adjustment, the operator fixes the amount of protrusion by tightening the fixing screw 54.

Even after the amount of protrusion of the sheath 7 is adjusted, the distal end of the needle tube 3 is positioned inside the distal end coil 71. The distal end of the needle tube 3 is present at any position of a position between the bending portion 105 and the angled portion 107b, a position inside the angled portion 107b, and a position inside the sloped portion 107a in the channel 107.

The connecting portion 73 of the sheath 7 is positioned closer to the proximal end side of the ultrasonic endoscope 100 than the proximal end 105b (refer to FIG. 4) of the bending portion 105 of the ultrasonic endoscope 100. Specifically, when the operator can optically observe the distal end of the sheath 7 via the ultrasonic endoscope 100, the connecting portion 73 is positioned closer to a proximal side than the proximal end 105b of the bending portion 105. In other words, when the operator can optically observe the distal end of the sheath 7 via the ultrasonic endoscope 100, only the distal end coil 71 and the needle tube 3 are disposed in a region from the proximal end 105b of the bending portion 105 to the distal end of the inserting portion 101.

Since only the distal end coil 71 and the needle tube 3 are disposed in the region from the proximal end 105b of the bending portion 105 to the distal end of the inserting portion 101, a decrease in the bending capability of the bending portion 105 can be prevented compared to when the proximal end coil 72 is positioned inside the bending portion 105.

Since the proximal end coil 72 is disposed in a region from the proximal end 105b of the bending portion 105 to the proximal end side, the meandering of the sheath 7 in this region can be suppressed. The proximal end coil 72 of the sheath 7 also has pliability such that the proximal end coil 72 can be deformed in the flexible tubular portion 106 so as to conform to deformation of the flexible tubular portion 106 which has flexibility and can be deformed into a bent shape. For this reason, when the operator moves forward and backward, or rotates the sheath 7 inside the channel 107, frictional resistance between the sheath 7 and the channel 107 is not excessively large and a response lag between an operation performed by the operator and a motion of the distal end of the sheath 7 is small.

Subsequently, the operator adjusts the maximum length of protrusion of the needle tube 3 by moving the stopper 61 while taking the distance between a target tissue T for biopsy and the distal end of the needle tube 3, and fixing the stopper 61 to the operation body 9 at a desired position, based on a result of observation via the ultrasonic scanning mechanism 104.

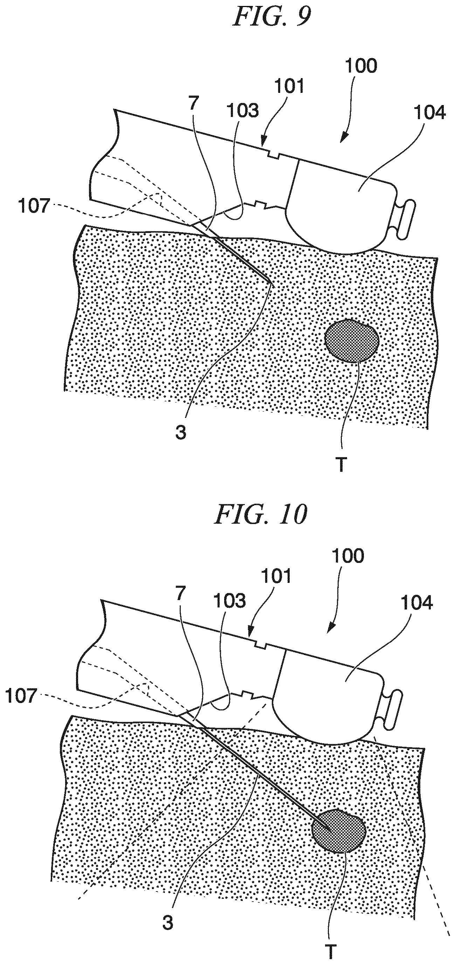

Subsequently, as shown in FIG. 8, the operator moves the needle slider 23 forward to a distal end side of the operation portion 8. As a result, as shown in FIG. 9, the needle tube 3 protrudes from the sheath 7. When the operator moves the needle slider 23 forward to the distal end side of the operation portion 8 in a state where the distal end of the needle tube 3 is positioned between the bending portion 105 and the angled portion 107b, the distal end of the needle tube 3 passes through the angled portion 107b and reaches the sloped portion 107a while being guided by the distal end coil 71 of the sheath 7.

When the distal end of the needle tube 3 is moved to the distal end side of the ultrasonic endoscope 100 from the angled portion 107b as a starting point, and also, when the distal end of the needle tube 3 is moved to the distal end side from the sloped portion 107a as a starting point, as described above, the distal end of the needle tube 3 protrudes from the distal end of the sheath 7 while being guided by the distal end coil 71.

As shown in FIG. 10, when the operator moves the needle slider 23 forward to the distal end side of the operation portion 8, the distal end of the needle tube 3 punctures the tissue and is pushed and moved forward to the target tissue T for biopsy. At this time, the operator can observe the needle tube 3, which is exposed to the outside above the surface of the tissue, via the optical image-capturing mechanism 103 and can observe a distal end side portion of the needle tube 3, which is inserted into the tissue, via the ultrasonic scanning mechanism 104.

The operator can observe an ultrasonic image, which is based on ultrasonic waves received by the ultrasonic scanning mechanism 104, via the ultrasonic observation unit 115 shown in FIG. 1. The operator adjusts the distal end of the needle tube 3 to reach the target tissue T for biopsy with reference to an image of the needle tube 3 clearly projected by the ultrasonic observation unit 115.

Subsequently, the operator pushes a tissue out, which is not a biopsy target jammed in the needle tube 3, via the stylet 27, and pulls the stylet 27 out of the inserting body 2 and the operation portion 8. Accordingly, a through hole extending from the distal end of the needle tube 3 to the proximal end of the needle slider 23 is formed. The operator connects a syringe or the like to the proximal end of the needle slider 23, suctions the inside of the needle tube 3 using the syringe or the like, and suctions and collects the cells of the target tissues T for biopsy through the distal end of the needle tube 3 using the syringe or the like.

When the necessary amount of cells or the like are collected, the operator moves the needle slider 23 backward to a proximal end side of the operation portion 8, and accommodates the distal end of the needle tube 3 inside the sheath 7. Accordingly, the needle tube 3 is pulled out of the tissue. When the needle tube 3 is pulled out of the tissue, the operator disconnects the slider lock 51 from the proximal end connector 108 of the operation portion 109 of the ultrasonic endoscope 100 and removes the biopsy needle 1 from the channel 107. Finally, the operator moves the ultrasonic endoscope 100 from a patient, and ends a series of treatments.

As described above, in the embodiment, since the meandering of the distal end coil 71 inside the channel 107 is suppressed and a decrease in the bending capability of the bending portion 105 is limited, the extension of the sheath 7 caused by the advancement and retraction of the needle tube 3 is limited, and the flexibility of the sheath 7 is obtained.

The distal end coil 71 and the proximal end coil 72 are designed to have different flexibility such that the distal end coil 71 has flexibility higher than that of the proximal end coil 72. For this reason, in the embodiment, when the biopsy needle 1 is mounted into the ultrasonic endoscope 100, a decrease in the bending capability of the bending portion 105 can be limited, and the extension and contraction of the sheath 7 inside the channel 107 in the direction of the center axis line or the meandering of the sheath 7 can be suppressed.

Since the resin coating 74 (disposed in close contact with the outer surface of the distal end coil 71) prevents the excessive movement of the spirals of the metal wire of the distal end coil 71 away from each other when the distal end coil 71 is bent and deformed, the jamming of the distal end of the needle tube 3 between the spirals of the metal wire can be prevented and the penetration of the needle tube 3 through the sheath 7 can be prevented.

Since the connecting portion 73 is positioned closer to the proximal end side of the ultrasonic endoscope 100 than a proximal end of the bending portion 105, even if the position of the sheath 7 is adjusted inside the channel 107, the proximal end coil 72 does not enter the inside of the bending portion 105. For this reason, no change in the bending capability of the bending portion 105 occurs before or after the position adjustment.

The sheath 7 may be configured such that the distal end coil 71 is provided over the total length of the sheath 7, and the proximal end coil 72 and the connecting portion 73 are not provided.

The sheath 7 may be configured such that the proximal end coil 72 is provided over the total length of the sheath 7, and the distal end coil 71 and the connecting portion 73 are not provided.

In thes cases, the resin coating 74 may be provided in the sheath 7 so as to cover the region of the distal end portion of the sheath 7 in which the distal end of the needle tube 3 may be positioned.

### [Examples]

Examples of the present invention will be shown.

In the examples of the present invention, the specific configuration of the proximal end coil 72 described in the embodiment will be shown, and the operation and the effects will be described. As a comparative example for showing the effects of the present invention, a coil with a different configuration will be shown.

**[Table 1]**

| | Example 1 | Example 2 | Comparative Example 1 |
|---|---|---|---|
| Outer Diameter [mm] | 1.46 | 1.56 | 1.67 |
| Inner Diameter [mm] | 0.95∼0.96 | 1.05∼1.06 | 1.15∼1.16 |
| Wire Diameter [mm] | 0.22∼0.24×0.6 | 0.22∼0.24×0.6 | 0.22∼0.24×0.6 |

Table 1 shows the dimensions of the coils which were prepared as Examples 1 and 2, and Comparative Example 1. Ten coil samples of each of Example 1, Example 2, and Comparative Example 1 were prepared. These coils have dimensions as shown in the embodiment.

**[Table 2]**

| | Example 1 | | Example 2 | | Comparative Example 1 | |
|---|---|---|---|---|---|---|
| | Initial Tension [N] | Spring Constant [N/mm] | Initial Tension [N] | Spring Constant [N/mm] | Initial Tension [N] | Spring Constant [N/mm] |
| Average Value | 5.79 | 0.97 | 5.47 | 0.73 | 4.01 | 0.64 |
| Maximum Value | 5.93 | 0.98 | 5.63 | 0.75 | 4.14 | 0.66 |
| Minimum Value | 5.54 | 0.96 | 5.30 | 0.71 | 3.87 | 0.63 |

Table 2 shows the initial tension and the spring constant of each of the ten coils for the examples and the comparative example. A resin coating described in the embodiment is provided in each of the coil samples, and an impact of the resin coating on the initial tension of the coil sample is negligible.

Each coil sample was inserted into a respiratory ultrasonic endoscope including a channel with an inner diameter of 2.0 mm or greater and 2.2 mm or less in a state where a 21G needle tube or a 22G needle tube was inserted into each coil sample, and the degree of extension of the coil sample in a direction of a center axis line of the coil sample and the frequency of occurrence of extension were investigated.

**[Table 3]**

| | Extension of Coil Sample | | | |
|---|---|---|---|---|
| | Frequency of Occurrence of Extension Exceeding 2.0 mm | Maximum Amount of Extension | Minimum Amount of Extension | Average Amount of Extension |
| Example 1 (22G Needle Tube) | 0[%] | 0[mm] | 0[mm] | 0[mm] |
| Example 2 (22G Needle Tube) | 0[%] | 1.0[mm] | 0[mm] | 0.2 [mm] |
| Example 2 (21G Needle Tube) | 0[%] | 2.0[mm] | 0[mm] | 0.4 [mm] |
| Comparative Example 1 (21G Needle Tube) | 40[%] | 5.0[mm] | 0[mm] | 1.55 [mm] |

When a sheath extends by 2 mm or more in a biopsy using an ultrasonic endoscope, the sheath pushes a tissue, and moves away from the ultrasonic endoscope, and thus, an ultrasonic image is disturbed in many cases. For this reason, the frequency of the occurrence of a defect (percent defective (%)) was investigated based on the assumption that the sheath extending by 2 mm or more is defective.

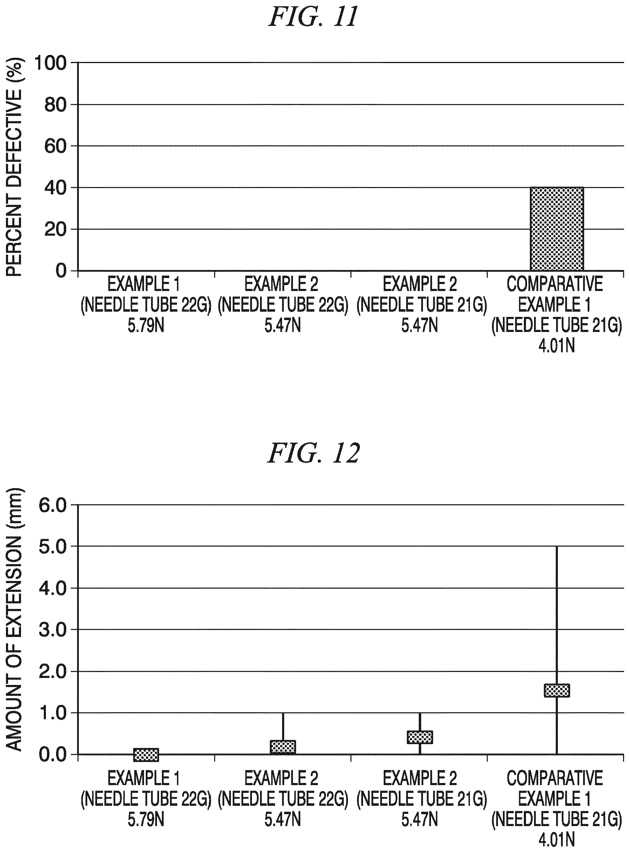

FIG. 11 is a graph showing a comparison between percent defectives in Example 1, Example 2, and Comparative Example 1 of the present invention. In FIG. 11, the horizontal axis represents the average initial tension of the coil of each of Example 1, Example 2, and Comparative Example 1, and the vertical axis represents the percent defective. As shown in FIG. 11 and Table 3, in Comparative Example 1, the frequency of occurrence (percent defective) of extension of the coil samples exceeding 2 mm was 40%.

As can be understood by Tables 2 and 3, the average amount of extension does not exceed 2 mm in any one of Examples 1 and 2, and Comparative Example 1. However, the average initial tension is between 4.01 N and 5.47 N, and FIG. 11 and Table 3 illustrate the occurrence of a defect and the non-occurrence of a defect. FIG. 12 is a graph showing a comparison between the amounts of extension of the sheaths in Example 1, Example 2, and Comparative Example 1 of the present invention. In FIG. 12, the horizontal axis represents the average initial tension of the coil of each of Example 1, Example 2, and Comparative Example 1 of the present invention, and the vertical axis represents the amount of extension of the coil. As shown in FIG. 12, it can be understood that a threshold value for the amount of extension is present between average initial tensions of 4.01 N and 5.47 N shown by rectangular solid shapes.

It can be understood by measurement results of the extension of the coil samples in Examples 1 and 2 and Comparative Example 1 that the initial tension value of the coil considerably affects the suppressing of the meandering of the sheath inside the channel, which leads to the extension of the sheath. The measurement results strongly indicate that a threshold at which the occurrence of a defect is zero is present between average initial tensions of 4.01 N and 5.47 N. When the initial tension of the coil forming the sheath exceeds 5 N, the extension of the sheath is remarkably limited compared to when the initial tension is approximately 4 N. That is, it can be said that the occurrence of extension is significantly decreased in the vicinity of an initial tension of 5 N, which is the boundary.

As described above, in an ultrasonic biopsy needle of the present invention, when the initial tension of the sheath exceeds 5.0 N under the condition that the ratio of the inner diameter of the channel of the ultrasonic endoscope to the outer diameter of the sheath of the ultrasonic biopsy needle is set to be in a range between 1.0:0.66 and 1.0:0.84, the occurrence of extension of the sheath can be limited. When the initial tension of the sheath is set to exceed 5.4 N, the occurrence of extension of the sheath can be further limited.

The occurrence of extension of a coil-shaped sheath satisfying the aforementioned conditions can be significantly limited compared to the related art. For this reason, when this sheath is used as a sheath of the ultrasonic needle for a respiratory organ, an operator can limit the extension of a distal end side of the sheath when a tissue is punctured, and stably and accurately perform a technique while observing an image.

The embodiment and the examples of the present invention have been described with reference to the drawings; however, the specific configuration is not limited to that in the embodiment, and a design change or the like can be made insofar as the design change does not depart from the purport of the present invention.

For example, a through hole (angled portion) bent or curved similar to the angled portion 107b may be formed in the distal end rigid portion 102, instead of the bent or curved cylindrical angled portion 107b. In this case, the angled portion 107b may be omitted.

The distal end coil 71 is welded to the proximal end coil 72 instead of using a cylindrical member as the connecting portion 73 through which the distal end coil 71 is connected to the proximal end coil 72. The position of the connecting portion 73 may be the position of the boundary at which the configuration of the wire is changed. For example, in a case where a wire-wound coil continues from the distal end to the proximal end of the sheath 7, the same difference in flexibility as that in the embodiment may be obtained by changing the sectional area, the sectional shape, the rigidness, or the like of the wire at the position of the connecting portion 73.

The biopsy needle 1 may not include the resin coating 74.

The embodiment of the present invention has been described with reference to the drawings; however, the specific configuration is not limited to that in the embodiment, and various changes can be made insofar as the changes do not depart from the purport of the present invention.

### [Industrial Applicability]

According to the embodiment, it is possible to provide an ultrasonic biopsy needle in which the extension of a sheath caused by the advancement and retraction of a needle tube is limited, and flexibility of the sheath is improved.

### [Reference Signs List]

- 1:: ULTRASONIC BIOPSY NEEDLE
- 3:: NEEDLE TUBE
- 7:: SHEATH
- 74:: RESIN COATING
- 8:: OPERATION PORTION
- 100:: ULTRASONIC ENDOSCOPE
- 102:: DISTAL END RIGID PORTION
- 103:: OPTICAL IMAGE-CAPTURING MECHANISM
- 105:: BENDING PORTION
- 105a:: DISTAL END OF BENDING PORTION
- 105b:: PROXIMAL END OF BENDING PORTION
- 106:: FLEXIBLE TUBULAR PORTION
- 107:: CHANNEL
- 107a:: SLOPED PORTION
- 107b:: ANGLED PORTION
- 107c:: CHANNEL TUBE
- 109:: OPERATION PORTION
- 110:: BENDING OPERATION MECHANISM

## Claims

1. An ultrasonic biopsy needle, comprising:
a sheath inserted through a channel of an ultrasonic endoscope;
a needle tube which is inserted through the sheath and capable of advancing and retracting in the sheath; and
an operation portion which is connected to a proximal end of the sheath and is provided to advance and retract the needle tube;
wherein the sheath includes a meandering suppress portion that is positioned more proximal than a proximal end of a bending portion of the ultrasonic endoscope when a distal end of the sheath is positioned in a visual field of an optical image-capturing mechanism of the ultrasonic endoscope, and
wherein the meandering suppressing portion is formed of a metal coil, the ratio of the inner diameter of the channel to the outer diameter of the coil is in a range from 1.0:0.66 to 1.0:0.84, and the initial tension of the coil is equal to or greater than 5.0 [N].

2. The ultrasonic biopsy needle according to claim 1,
wherein the sheath is formed of the coil in the entire length of the sheath.

3. The ultrasonic biopsy needle according to claim 1,
wherein the coil includes a metal wire, and a resin coating with which the metal wire is coated.

4. The ultrasonic biopsy needle according to claim 3,
wherein the spring constant of the coil is 0.7 N/mm.

5. The ultrasonic biopsy needle according to claim 1,
wherein the channel has an inner diameter of equal to or greater than 2.0 mm and equal to or less than 2.2 mm,
wherein a sloped portion is formed on a distal end side of the channel such that the angle of the sloped portion is fixed in order for the ultrasonic biopsy needle to protrude from the channel while being inclined with respect to a vibrator of the ultrasonic endoscope, and
wherein the maximum amount of movement of the needle tube with respect to the sheath is equal or greater than 5% of the total length of the sheath.
